# EUROPEAN PATENT APPLICATION

(11) **EP 4 809 958 A1**
(43) Date of publication of application: **23.09.2026**
(21) Application number: 24890434.4
(22) Date of filing: 18.10.2024
(51) Int. Cl.: A61B 34/20, A61B 34/10, A61B 17/00, A61B 90/00, A61B 1/317

(54) **NAVIGATION METHOD FOR ENDOSCOPIC SPINE SURGERY, ELECTRONIC DEVICE, AND NAVIGATION SYSTEM**

(30) Priority: 13.11.2023 CN 202311503182
(71) Applicant: Changzhou Kanghui Medical Innovation Co., Ltd., Changzhou Jiangsu 213022 (CN)
(72) Inventor: LV, Xin, Shanghai 200126 (CN); MA, Zhaoxuan, Shanghai 201114 (CN)
(74) Representative: Viering, Jentschura & Partner mbB Patent- und Rechtsanwälte
(86) International application number: PCT/CN2024/125820
(87) International publication number: WO 2025/103076

(57) **Abstract**

A navigation method and navigation system for endoscopic spine surgery. The method comprises the following steps: a three-dimensional image acquisition step: acquiring a three-dimensional image of at least a part of a spine; an image acquisition step: acquiring an image of an endoscope in real time; an orientation acquisition step: acquiring the position and direction of the tip of the endoscope in real time; an image segmentation step: segmenting the three-dimensional image on the basis of the position and direction of the tip of the endoscope; a fusion step: fusing the segmented three-dimensional image and the image of the endoscope; and a display step: displaying a fused image. According to the method, in an endoscopic spine surgery scenario, augmented reality fusion of a real-time endoscopic field of view and the three-dimensional image around the endoscope is achieved, providing, in spine surgery, doctors with more visual orientation information of the tip of the endoscope and of the tip of a surgical tool in a spine structure, thereby avoiding misoperation to a great extent and increasing the reliability of the spine surgery.

## Description

### Technical Field

The present invention relates to the technical field of medical device, in particular to surgical navigation systems, and, more specifically, relates to an image fusion-based navigation method and navigation system for endoscopic spine surgery.

### Background Art

Endoscopic spine surgery technology is well received by patients as well as minimal invasive spinal surgeons due to its clinical advantages such as minimal invasiveness, reduced intraoperative blood loss and shortened length of hospital stay. However, conventional endoscopic spine surgery typically has an extremely steep learning curve, imposing high requirements on anatomical knowledge and spatial imagination capability of surgeons, which greatly limits the clinical promotion of endoscopic spine surgery technology.

In conventional endoscopic surgery, surgical observation, instrument operation and control are performed merely under endoscopic field of view. With only endoscopic view available, surgeons lack osseous structures as reference, making it difficult to identify soft tissue structures in the two-dimensional planar endoscopic images. Surgeons are prone to losing the global field of view and spatial orientation, resulting in poor hand-eye coordination. Consequently, the endoscope may fail to reach the lesion accurately or decompression may be insufficient, leading to failure in achieving predetermined surgical objectives.

The aforementioned drawbacks become more prominent in endoscopic spine surgery, particularly in transforaminal endoscopic surgery. In endoscopes adopted for spine surgery, channels of camera and instrument are integrated into a single endoscopic insertion tube. When a surgeon rotates the endoscope to adjust the endoscopic camera for observing a target position, the surgical instrument rotates synchronously with the endoscope, changing the spatial orientation of the surgical instrument relative to the spine. This makes it more difficult for surgeons to perceive the global orientation of the surgical instrument relative to the spine and increases the risk of misoperation.

Furthermore, surgical instruments used in minimal invasive endoscopic spine surgery include instruments with distal inclined angles, flexible-shaft instruments, instruments with distal expandable structures, and the like. Therefore, actual spatial orientation of the distal end of the instruments, i.e. the working end, cannot be acquired by surgeons relying solely on two-dimensional endoscopic images, which also easily causes misoperation and surgical errors.

Conventional navigation technologies are mostly applied in spinal internal fixation surgery for real-time tracking and positioning of instruments and implants relative to the patient's anatomical structure during pedicle screw insertion, assisting surgeons in achieving precision, safety and minimal invasiveness in clinical practice. Among them, optical navigation has gained increasing clinical attention in spine surgery due to its high positioning accuracy, strong anti-interference capability and low application cost.

### Summary of the Invention

The present invention aims to solve at least one of the above problems and defects existing in the prior art as well as other technical problems.

In one aspect, the present invention provides a navigation method for endoscopic spine surgery, the method comprising the following steps:
a three-dimensional image acquisition step: acquiring a three-dimensional image of at least a portion of the spine;
an image acquisition step: acquiring real-time endoscopic images;
an orientation acquisition step: acquiring real-time position and direction of an endoscope distal end;
a three-dimensional image cutting step: cutting the three-dimensional image according to the position and direction of the endoscope distal end;
a fusion step: fusing the cut three-dimensional image with the endoscopic image; and a display step: displaying the fused image.

In the navigation method for endoscopic spine surgery, augmented reality fusion is performed between real-time endoscopic images and global three-dimensional images, which is particularly beneficial for navigation in endoscopic spine surgery. As described in the background, due to the special structural design of adopted spinal endoscopes that makes surgical instruments rotate synchronously with the endoscopic camera and the particularity of adopted instrument structures, orientation of the surgical instruments distal end often vary and are difficult to track. Surgeons are more likely to lose spatial orientation when observing endoscopic images and struggle to determine the position and direction of the instrument distal end. The present invention realizes augmented reality fusion between the real-time endoscopic field of view (where the instrument distal end is visible) and three-dimensional images surrounding the endoscope via navigation system in endoscopic spine surgery. Intuitive orientation information of the endoscope distal end and the surgical instrument distal end relative to spinal anatomical structures is provided for surgeons during spine surgery, which can substantially avoid misoperation and improve the reliability of spine surgery.

According to an embodiment, in the three-dimensional image cutting step, the three-dimensional image is cut into a cylindrical shape; diameter of the cylinder is determined according to input from an operator, so as to define the cutting range of the three-dimensional image and obtain a cut three-dimensional image within a display range needed by the operator.

The exemplary embodiment allows an operator to select freely the viewing range of the three-dimensional image as needed, for example, enlarging or reducing the cylinder diameter by operating a zoom icon described below, thereby achieving free scaling of the viewing range of the three-dimensional image. This offers particular benefits for endoscopic spine surgery. The field of view of an endoscope is extremely narrow; even experienced spinal surgeons may struggle to identify osseous structures within the limited endoscopic coverage area, or encounter unfamiliar viewing angles of osseous structures. Surgeons sometimes need to view a wider or global three-dimensional image to determine the precise position and lens direction of the endoscope relative to the patient's anatomical structure and make corresponding adjustments. At other times, magnified three-dimensional images (with a correspondingly reduced display range) are needed to observe fine details of anatomical structures. Frequent switching between the two viewing modes is often necessary. In the exemplary embodiment, the cutting range of the three-dimensional image can be adjusted through interaction by an operator to meet real-time observation demands, especially via the provided zoom function. The embodiment breaks the constraint of the narrow endoscopic field of view and greatly facilitates observation during endoscopic spine surgery.

Moreover, as aforementioned, surgeons are prone to spatial disorientation when observing endoscopic images due to the special structure of spinal endoscopes and the particularity of surgical instruments. This solution not only realizes augmented reality fusion between the real-time endoscopic field of view (with the instrument distal end visible) and surrounding three-dimensional images, but also allows adjustment and selection of the display range of the three-dimensional image as needed. It enables better observation of the spatial positional relationship between the endoscope distal end, the instrument distal end and surrounding three-dimensional images (corresponding to the patient's osseous structures), thereby significantly reducing misoperation risks and enhancing the reliability of spine surgery.

According to an embodiment, the fusion step further comprises scaling the size of the endoscopic image correspondingly to three-dimensional images with different cutting ranges prior to fusion.

The exemplary embodiment enables the endoscopic image to occupy an appropriate proportion in the final fused image. For instance, when the cutting range of the three-dimensional image is small with a correspondingly enlarged display scale, surgeons can observe osseous structures beneath soft tissues within the endoscopic field of view. When the cutting range of the three-dimensional image is large with a correspondingly reduced display scale, the endoscopic image is scaled down synchronously, allowing surgeons to observe peripheral osseous structures outside the endoscopic field of view, facilitating determination of endoscope position and direction and subsequent operational adjustment.

According to an embodiment, the orientation of the endoscope distal end is defined as the optical axis direction of an endoscopic camera; cutting starts from the position of focal plane of the endoscopic camera, extends along the optical axis direction, and penetrates the entire depth of the three-dimensional image. Cutting through the full depth of the three-dimensional image can completely present osseous structures of the spine within and around the endoscopic field of view.

According to an embodiment, the endoscope distal end is formed with a sloped surface inclined relative to the longitudinal axis of the endoscope, and the optical axis direction of the endoscope is perpendicular to the sloped surface.

According to an embodiment, in the three-dimensional image acquisition step, a preoperative three-dimensional image of at least a portion of the spine is registered with the patient's pose under a navigation coordinate system.

According to an embodiment, a distortion processing step is further included prior to the fusion step. In the distortion processing step, one of the following three approaches is selectable according to input from an operator:
performing distortion correction on the acquired endoscopic image to at least partially eliminate distortion effects of the endoscopic camera, such that the distortion-corrected endoscopic image matches the cut three-dimensional image;
performing distortion processing on the cut three-dimensional image according to endoscopic camera parameters, such that the distortion-processed three-dimensional image matches the real-time acquired endoscopic image; or
simultaneously performing distortion correction on the endoscopic image and distortion processing on the three-dimensional image to match the distortion degrees therebetween, and thereafter, correspondingly to the distortion processing step, the fusion step comprises: fusing the distortion-corrected endoscopic image with the cut three-dimensional image; or fusing the real-time acquired endoscopic image with the distortion-processed three-dimensional image; or fusing the distortion-corrected endoscopic image with the distortion-processed three-dimensional image.

This embodiment provides multiple choices for surgeons to make flexible selection according to personal preferences. During distortion processing, endoscopic camera distortion parameters are adopted to calibrate endoscopic images to eliminate camera distortion effects, generating a view consistent with a real perspective scene and obtaining a normal-field image that visually matches the undistorted three-dimensional image for easy observation. Alternatively, endoscopic camera parameters can be used to perform distortion processing on the cut three-dimensional image, endowing the three-dimensional image with the same distortion characteristics as the endoscopic image to achieve visual matching with the actual distorted endoscopic image, which is more convenient for surgeons accustomed to observing distorted endoscopic images. In addition, simultaneous distortion correction of endoscopic images and distortion processing of three-dimensional images can be performed to match the distortion degrees of both.

According to an embodiment, in the fusion step, one of the following two fusion modes can be selected according to input from an operator, or images obtained by both fusion modes can be presented simultaneously:
performing fusion by overlaying the cut three-dimensional image underneath the real-time acquired endoscopic image; and
performing fusion by embedding the real-time acquired endoscopic image into the acquired three-dimensional image at position and direction corresponding to the endoscope distal end.

This embodiment provides surgeons with two optional fusion modes. The first fusion mode is suitable for surgeons accustomed to the native endoscopic field of view; the second mode facilitates global anatomical observation. Surgeons can make flexible selections according to personal preferences.

According to an embodiment, in the display step, three-dimensional images located behind the endoscope distal end are blurred and displayed.

This embodiment provides surgeons with more intuitive images to better perceive the advancing direction of the endoscope.

According to an embodiment, the navigation method further comprises an endoscope distal end calibration step for calibrating the positional relationship between the endoscope distal end and a first tracker disposed on the endoscope; the endoscope distal end calibration step is implemented via a calibration tool without acquiring images through the endoscope, reducing the workload of image processing.

In another aspect, the present invention further provides a computer-readable storage medium storing a computer program, wherein the program, when executed by a processor, implements the steps of the method according to any of the above embodiments.

In another aspect, the present invention further provides a control device, comprising a memory, a processor, and a computer program stored in the memory and executable by the processor, wherein the processor executes the program to implement the steps of the method according to any of the above embodiments.

In another aspect, the present invention further provides an electronic device for navigation in endoscopic spine surgery, the electronic device comprising a display apparatus and a processor, the processor being provided with a data interface, wherein the data interface is connectable to an endoscope, enabling the processor to acquire real-time endoscopic images and real-time position and direction of the endoscope distal end; and wherein the data interface further enables the processor to acquire a three-dimensional image of at least a portion of the spine; and wherein the processor displays a fused image on the display apparatus during operation, the fused image being obtained by fusing a three-dimensional image corresponding to the position and direction of the endoscope distal end with the endoscopic image.

In another aspect, the present invention further provides a navigation system for endoscopic spine surgery, the navigation system comprising a tracking device, a display apparatus and a processor; the tracking device is configured to track a first tracker disposed on an endoscope and a second tracker disposed at a target anatomical site of a patient; the processor executes the steps of the method according to any of the above embodiments during operation, and realizes display in the navigation method via the display apparatus.

In still another aspect, the present invention further provides a navigation system for endoscopic spine surgery, the navigation system comprising a tracking device and the electronic device according to any of the embodiments.

According to an embodiment, the navigation system further comprises one or more of the endoscopes, the first tracker and the second tracker.

According to an embodiment, the navigation system further comprises a calibration tool for calibrating the position of the endoscope distal end relative to the first tracker; the calibration tool is provided with a plurality of calibration holes, the plurality of calibration holes having bottom surfaces with different inclination angles and/or different hole diameters, so as to calibrate endoscopes with different inclination angles of distal sloped surface and/or different tube diameters respectively.

### Brief Description of the Drawings

The present invention is described in detail below with reference to the accompanying drawings and exemplary embodiments.
FIG. 1 is a flowchart of a navigation method for endoscopic spine surgery according to an exemplary embodiment of the present invention.
FIG. 2 is a schematic principle diagram of a navigation system for endoscopic spine surgery according to an exemplary embodiment of the present invention.

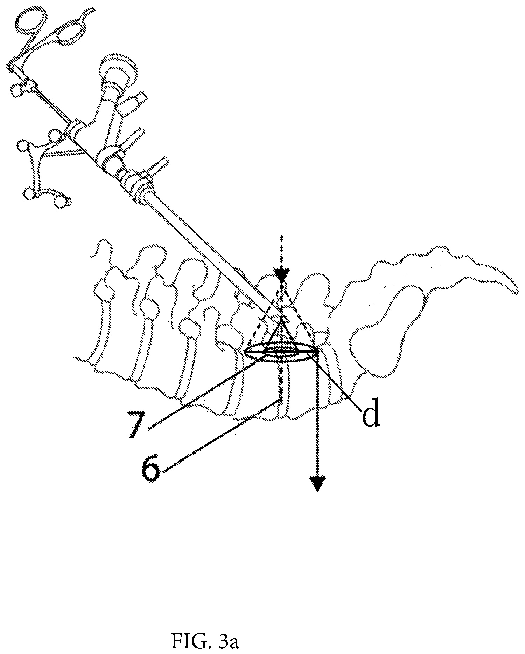

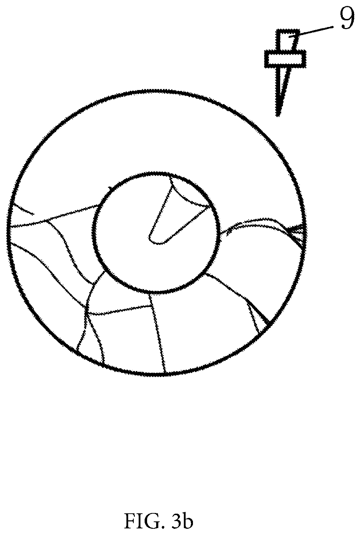

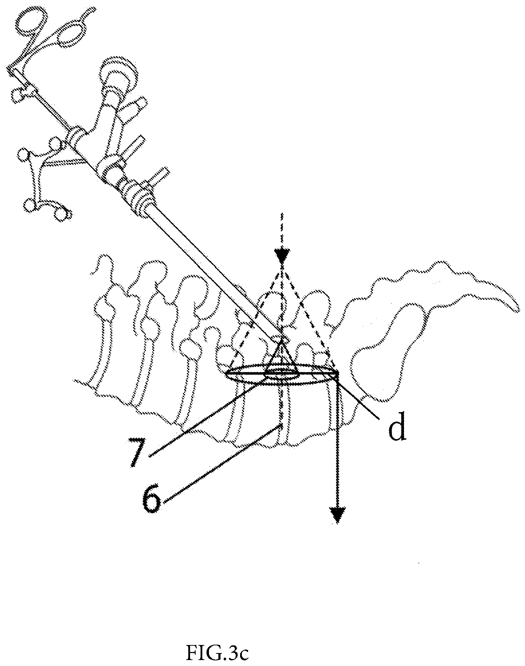

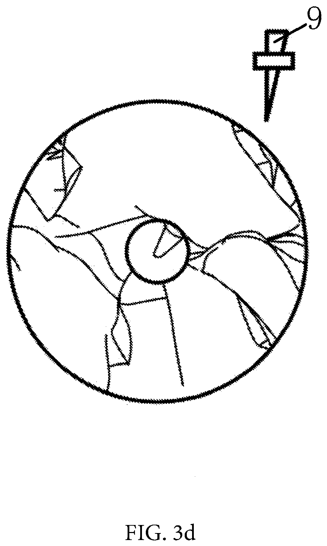

FIGS. 3a to 3d are schematic principle diagrams illustrating three-dimensional image cutting and fusion of the cut three-dimensional image with an endoscopic image according to requirement from an operator, wherein FIG. 3a is a schematic diagram of cutting a three-dimensional image within a small range, FIG. 3b is an image obtained by fusing the three-dimensional image cut in FIG. 3a with an endoscopic image, FIG. 3c is a schematic diagram of cutting a three-dimensional image within a large range, FIG. 3d is an image obtained by fusing the three-dimensional image cut in FIG. 3c with an endoscopic image.
FIGS. 4a and 4b schematically illustrate images obtained by two fusion modes.
FIG. 5 is a schematic diagram of an exemplary display apparatus.

It shall be noted that the accompanying drawings are merely schematic, and only illustrate parts or steps necessary for demonstrating the present invention, while other parts or steps may be omitted or briefly mentioned. The present invention may include other components or steps besides those illustrated in the accompanying drawings.

### Detailed Description of the Embodiments

The technical solutions of the present invention are described in further detail below through embodiments in conjunction with the accompanying drawings. The following description of the embodiments of the present invention with reference to the accompanying drawings is intended to demonstrate the general concept of the present invention and shall not be construed as a limitation thereto.

Hereinafter, specific steps of the navigation method for endoscopic spine surgery and the corresponding navigation system thereof are described by way of example. In the following detailed description, numerous specific details and steps are set forth to provide a comprehensive understanding of the embodiments. It should be understood, however, that one or more other embodiments may be practiced without these specific details.

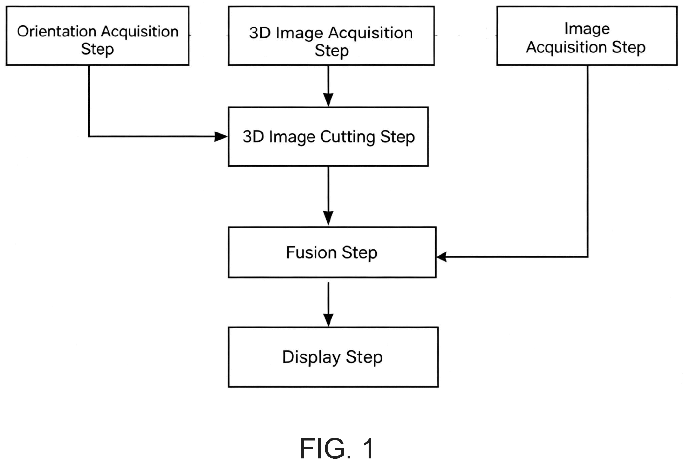

FIG. 1 shows a flowchart of a navigation method for endoscopic spine surgery according to an exemplary embodiment. The method includes a three-dimensional image acquisition step of acquiring a three-dimensional image of at least a portion of the spine, an image acquisition step of acquiring real-time endoscopic images, an orientation acquisition step of acquiring real-time position and direction of the spinal endoscope distal end, a three-dimensional image cutting step of cutting the three-dimensional image according to the position and direction of the endoscope distal end, a fusion step of fusing the cut three-dimensional image with the endoscopic image, and a display step of displaying the fused image. It shall be understood that the sequential listing of the above steps in the flowchart of FIG. 1, the claims and the present description does not imply a fixed chronological order therebetween. For example, the three-dimensional image acquisition step may be performed before, after, or simultaneously with the image acquisition step and/or the orientation acquisition step, and the image acquisition step may also be performed before, after, or simultaneously with the orientation acquisition step.

As shown in FIG. 1, in the navigation method provided by the present invention, a three-dimensional image is acquired and real-time endoscopic images are captured; the navigation system is utilized to acquire real-time position and direction of the endoscope distal end, and the three-dimensional image is fused and displayed with real-time endoscopic images in position and direction corresponding to the endoscope distal end. The augmented reality combination of actual real-time images and global three-dimensional images offers particular advantages for navigation in endoscopic spine surgery. As described in the background, surgeons are prone to spatial disorientation and difficulty in determining the position and direction of the instrument distal end when observing endoscopic images due to the special structure of spinal endoscopes and the particularity of surgical instruments. The present invention realizes an augmented reality surgical solution integrating three-dimensional images and real-time endoscopic field of view (with the instrument distal end visible) in combination with a navigation system, which provides intuitive and effective intraoperative information to surgeons with substantially improved surgical reliability.

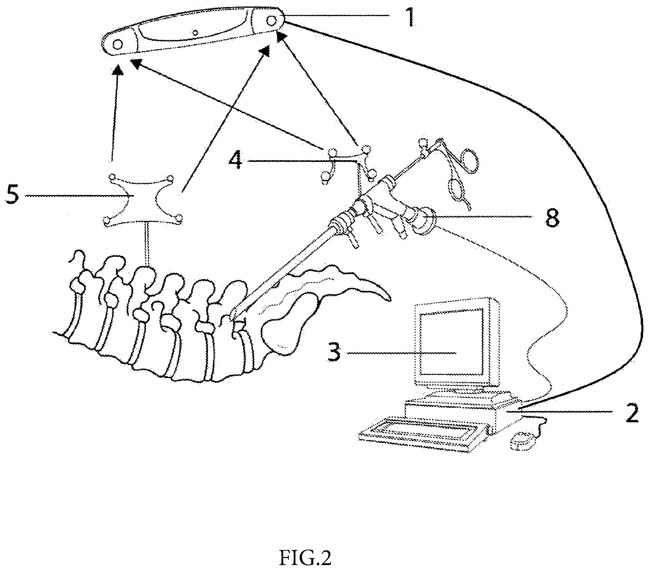

In a specific embodiment, as shown in FIG. 2, the navigation system for endoscopic spine surgery includes a tracking device 1, a control device 2 and a display apparatus 3. Tracking device 1 may be an optical tracking device (e.g., an NDI navigator); correspondingly, a first optical tracker 4 may be disposed on the endoscope, and a second optical tracker 5 can be disposed at a target anatomical site of the patient. Those skilled in the art shall appreciate that tracking device 1 may also adopt other types, such as an electromagnetic tracking device. As a specific example, control device 2 may be a general-purpose computer, a dedicated computer, an embedded processor, or any other suitable programmable data processing equipment such as a single-chip microcomputer or a chip. Control device 2 may include a processor and a memory for storing programs, or only a processor externally connected to memory storing programs. In other words, the control device at least includes a processor. Control device 2 (or the processor) and display apparatus 3 may be integrated or separately arranged. The control device or the processor is provided with a data interface connectable to the endoscope, enabling the control device/processor to acquire real-time endoscopic images and real-time position and direction of the endoscope distal end. The data interface may also be connected to, for example, CT equipment, allowing the processor to acquire a three-dimensional image of at least a portion of the spine via the data interface. As an exemplary implementation, one or more of the endoscopes, the first tracker and the second tracker may also be regarded as part of the navigation system.

Endoscope 8 is schematically shown in FIG. 2, which may be, for example, a transforaminal endoscope. Typically, the endoscope distal end penetrates the patient's tissue and osseous structures for observation and/or operation, while the proximal end (opposite to the distal end and close to the operator) is located outside the patient's body for manipulation by the operator. In the navigation system, the first tracker suitable for being tracked by tracking device 1 is usually disposed at the extracorporeal proximal end of the endoscope, such that the navigation system can acquire position and direction of first tracker 4 on endoscope 8 in the navigation coordinate system in real time. In the present invention, the endoscopic camera is arranged at the distal end of the endoscopic insertion tube. By calibrating the relative positional relationship between the distal end of the endoscopic insertion tube and first tracker 4 on the endoscope, the position and direction of the endoscopic camera in the navigation coordinate system can be further determined.

Specifically, prior to performing endoscopic spine surgery and navigation, the relative positional relationship between the endoscopic camera and the first tracker may be calibrated. Preferably, in this embodiment, the calibration is implemented via a calibration tool (not shown in the figure) without acquiring images through the endoscope, reducing the workload of image processing. The navigation system of the present invention may optionally include the calibration tool. The calibration tool is provided with a plurality of calibration holes having bottom surfaces with different inclination angles and/or different hole diameters, so as to calibrate endoscopes with different inclination angles of distal sloped surface and/or different tube diameters respectively. Specifically, the calibration tool further includes a third tracker fixed on its bracket, and the navigation system can acquire the position of the third tracker in the navigation coordinate system. The positional relationship of each calibration hole relative to the third tracker is known according to the design dimensions of the calibration tool, whereby the positions of the calibration holes in the navigation coordinate system are determined. The distal end of the insertion tube of the endoscope to be calibrated is inserted into a matched calibration hole, and the sloped surface of the insertion tube distal end is attached to the inclined bottom surface of the calibration hole to achieve positioning of the endoscope. Since the navigation system can acquire the position of the first tracker on the endoscope at the same time in the navigation coordinate system, the relative positional relationship between the distal end of the endoscopic insertion tube and the first tracker can be determined, completing the calibration process.

Intraoperatively, real-time acquisition of the position of the first tracker on the endoscope via tracking device 1 enables indirect real-time acquisition of the position and direction of the endoscope distal end, i.e., the position and direction of the endoscopic camera. The aforementioned orientation acquisition step in the navigation method of the present invention is implemented in this manner.

In this specific embodiment, calibration of endoscopic camera parameters may also be performed prior to endoscopic spine surgery and navigation operation. Exemplarily, camera parameter calibration may use a calibration plate; for example, the endoscope captures images of the calibration plate at different position and direction angles, and image data and calibration plate specifications are recorded to calibrate camera parameters, distortion matrixes, and/or rotation and displacement vectors of the endoscopic camera. This can be exemplarily implemented via the calibrateCamera calibration function provided by OpenCV (a cross-platform computer vision library for camera-related image processing). The calibrated endoscopic camera parameters are adopted in the subsequent distortion correction process.

According to the navigation method of the present invention, in the three-dimensional image acquisition step, a preoperative three-dimensional image of at least a portion of the spine, such as a preoperative CT image, is acquired and registered to the intraoperative navigation coordinate system. Alternatively, a three-dimensional image acquired intraoperatively via CBCT may be adopted.

Subsequently, as shown in FIGs. 3a to 3d, the acquired three-dimensional image is cut according to the position and direction of the endoscope distal end, so as to fuse real-time endoscopic images with three-dimensional images of surrounding regions, implementing the three-dimensional image cutting step and the fusion step in the navigation method of the present invention. In the embodiments shown in FIGs. 3a and 3c, the endoscope distal end, i.e., the distal end of the insertion tube, is formed with a sloped surface relative to the longitudinal axis of the endoscope; optical axis direction 6 of the endoscopic camera is perpendicular to the sloped surface and forms a certain included angle with the axial direction of the insertion tube. The cutting position and direction of the three-dimensional image vary according to the position and optical axis direction of the endoscope distal end. As shown in FIGs. 3a and 3c, the viewing direction of the three-dimensional image (direction indicated by the arrow in FIGs. 3a and 3c) is coaxial with the optical axis, i.e., coaxial with the viewing direction of the endoscope. In other words, the three-dimensional image in the fused image is presented from the viewing perspective of the endoscope. In FIGS. 3a and 3c, the end face of the cut cylinder is perpendicular to optical axis direction 6.

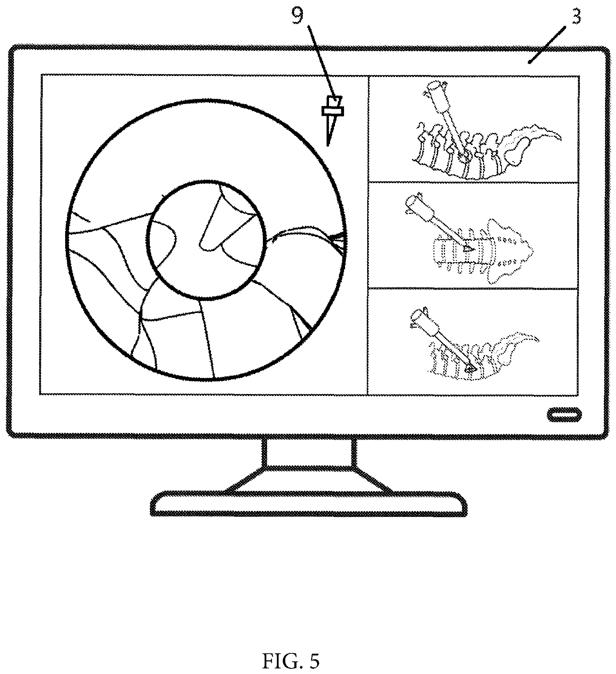

Preferably, an operator such as a surgeon can select the range of the three-dimensional image to be fused as needed; in other words, the cutting range of the three-dimensional image can be adjusted according to real-time input from the operator. For example, zoom icon 9 (schematically shown in FIGs. 3b, 3d and FIG. 5) may be arranged on the display interface of the display apparatus. The operator can manipulate the zoom icon via an input device according to the desired viewing range of the three-dimensional image. Input devices include but are not limited to a mouse, a keyboard, a touch screen and the like. When the operator intends to view a three-dimensional image of spinal structures within a larger range around the endoscope (corresponding to a reduced display scale, by operating the zoom icon toward a reducing end), the diameter of the cut three-dimensional cylindrical volume (preferably a cylinder in the illustrated embodiment) is increased as shown in FIG. 3c. The fused image obtained is shown in FIG. 3d, presenting a larger range of three-dimensional spinal structures. When the operator intends to enlarge the view for observing fine details of spinal structures (corresponding to an enlarged display scale, by operating the zoom icon toward an enlarging end), the diameter of the cut cylinder is decreased as shown in FIG. 3a. The fused image obtained is shown in FIG. 3b, enabling clearer observation of fine details of three-dimensional spinal structures. This solution facilitates free scaling of the viewing range of the three-dimensional image by the operator as needed, which is particularly beneficial for spine surgery. Surgeons sometimes need to view a larger or global three-dimensional image to determine the position and direction of the endoscope and adjust operations accordingly; during specific procedures, observation of fine details of three-dimensional structures beneath soft tissues within the endoscopic field of view is also needed. The zoom function realized by adjusting the cutting range of the three-dimensional image according to real-time input from the operator greatly facilitates operation in endoscopic spine surgery. Those skilled in the art shall appreciate that the input from an operator for defining the three-dimensional image cutting range is not limited to operation of a zoom icon; other approaches are also applicable, such as providing multiple optional preset display ranges for selection or allowing numerical input from the operator to define the cutting range, which can be implemented via input components on the display interface such as the aforementioned zoom icon, tabs for selecting different three-dimensional image display ranges, or dialog boxes for numerical input.

Preferably, the cutting of the three-dimensional image penetrates the entire depth thereof, which is particularly applicable to spinal surgical since the acquired three-dimensional spinal image mainly involves osseous structures without interference from other soft tissues. Cutting through the full depth of the three-dimensional image can completely present relevant spinal anatomical structures. In this specific embodiment, as shown in FIGs. 3a and 3c, direction of the endoscope distal end is defined as optical axis direction 6 of the endoscopic camera; cutting of the three-dimensional image starts from focal plane 7 of the endoscopic camera, and a cylinder with diameter d is cut along optical axis direction 6 to penetrate the entire depth of the three-dimensional image.

Preferably, in the fusion step, the size of the endoscopic image is scaled synchronously with the three-dimensional image prior to fusion. By comparing FIGs. 3a, 3b with FIGs. 3c, 3d, when the cutting range shown in FIG. 3c is larger than that in FIG. 3a, the display range of the three-dimensional image in the fused image shown in FIG. 3d is larger with a correspondingly smaller display scale, and the endoscopic image is scaled down synchronously, enabling the endoscopic image to occupy an appropriate proportion in the overall fused image (refer to the corresponding fused image shown in FIG. 3d).

Endoscopic images have inherent distortion effects generated by the endoscopic camera. To achieve better matching between endoscopic images and three-dimensional images and facilitate operator observation, distortion correction may be performed on endoscopic images, or distortion processing may be applied to three-dimensional images to match the distortion characteristics of endoscopic images, or both endoscopic image distortion correction and three-dimensional image distortion processing may be performed to align the distortion degrees therebetween. In the distortion processing step of the navigation method of the present embodiment, any one of the three aforementioned modes is selectable according to input from an operator, allowing flexible selection by surgeons based on personal preferences. In the distortion processing step, endoscopic camera parameters calibrated in advance are adopted to calibrate endoscopic images and eliminate camera distortion effects. Endoscopic image acquisition can be implemented via a high-definition image capture card, which converts endoscopic video signals into a video stream, and frame-by-frame distortion correction is performed to obtain calibrated endoscopic images. Alternatively, according to selection by a surgeon, instead of calibrating endoscopic images, the pre-calibrated endoscopic camera parameters are used to perform distortion processing on the cut three-dimensional image, endowing the three-dimensional image with the same distortion characteristics as the endoscopic image. This mode is more convenient for surgeons accustomed to observing distorted endoscopic images. Alternatively, a third mode may be adopted, wherein both endoscopic image distortion correction and three-dimensional image distortion processing are performed to match the distortion degrees of both.

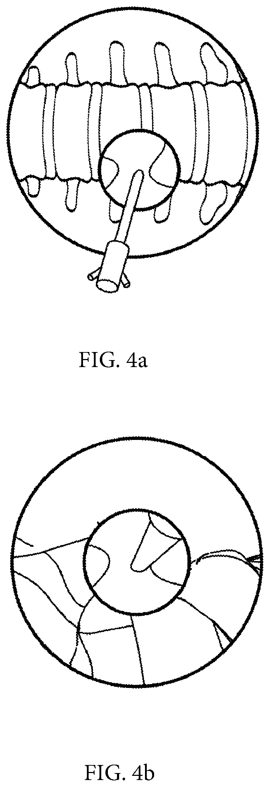

The specific embodiment of the navigation method of the present invention further provides surgeons with two optional image fusion modes. Specifically, in the fusion step, the method allows selection of one of the following two fusion modes according to input from an operator: the first fusion mode is performed by overlaying the cut three-dimensional image underneath the real-time acquired endoscopic image, and FIG. 4b shows an exemplary image obtained by this fusion mode. The second fusion mode is performed by embedding the real-time acquired endoscopic image into the acquired three-dimensional image at a position and viewing angle corresponding to the endoscope distal end, and FIG. 4a shows an exemplary image obtained by this fusion mode. The first fusion mode, also referred to as endoscopic view fusion, retains the native endoscopic field of view while overlaying a 3D anatomical view at the bottom layer, maintaining surgeons' preferred endoscopic operation mode while providing a global macroscopic view. The second fusion mode, also referred to as 3D global view fusion, embeds the real-time endoscopic image into the three-dimensional image at the position of the endoscope distal end from the viewing angle defined by the optical axis direction of the endoscopic camera acquired by the navigation system, facilitating surgeons' global anatomical observation. Those skilled in the art shall appreciate that images obtained by both fusion modes may be presented simultaneously on the display apparatus.

In the display step, three-dimensional images located behind the endoscope distal end (i.e., closer to the proximal end relative to the endoscope distal end) may be blurred for display according to selection by a surgeon, providing surgeons with more intuitive visual feedback to better perceive the advancing direction of the endoscope.

The present invention further provides an electronic device including a display apparatus 3 and a processor; in the specific embodiment shown in FIG. 2, the processor is integrated into control device 2, i.e., the illustrated host. Those skilled in the art shall appreciate that the processor and the display apparatus may be integrated or separate devices. Control device 2 or the processor is provided with a data interface. The control device is electrically connected to the endoscope via the data interface to acquire real-time endoscopic images and real-time position and direction of the endoscope distal end. The data interface may also be connected to CT equipment (e.g., CBCT, it shall be understood by those skilled in the art that connection is not limited to CT equipment, and connection to a storage device storing three-dimensional image data is also applicable) to acquire a three-dimensional image of at least a portion of the spine via the data interface. When the processor executes a computer program (the program may be stored in a memory included in the control device or other external storage media), a fused image is displayed on display apparatus 3 (the left image in FIG. 5). The fused image is obtained by fusing a three-dimensional image corresponding to the position and direction of the endoscope distal end with the endoscopic image. The fused three-dimensional image may be obtained by cutting the three-dimensional image according to the position and direction of the endoscope distal end as described above (preferably enlarging or reducing the cutting range of the three-dimensional image according to real-time operation by an operator on the zoom icon).

The display interface may be provided with input components, wherein the display range of the three-dimensional image in the fused image is determined by input from an operator via the input components. The input components include, for example, a zoom icon, and the display range of the three-dimensional image in the fused image is enlarged or reduced in real time in response to operator operation on the zoom icon. Exemplarily, input from the operator for defining the display range of the three-dimensional image may also be implemented via components on the display interface for selecting different preset three-dimensional image display ranges (e.g., tabs), or other components such as dialog boxes for inputting numerical values of cylindrical diameter.

As an exemplary embodiment, the display interface of display apparatus 3 may further present a three-dimensional navigation view, including one or more views showing the spatial positional relationship between the endoscope and its field angle relative to the patient's anatomical structure on fitted two-dimensional perspective sections, sagittal sections, coronal sections, axial sections, sections along the instrument axis, and the like. For this purpose, preoperative CT images or intraoperative CBCT three-dimensional images are acquired for navigation in the three-dimensional image acquisition step. Some of these views are exemplarily shown in FIG. 5. Each view on the display interface is updated in real time with changes in the position and direction of the endoscope distal end, enabling the operator to acquire comprehensive navigation information. Those skilled in the art shall appreciate that views displayed on the display interface are not limited to those described above and illustrated in FIG. 5, and other sections and orientation views may be displayed as needed.

Exemplarily, the display interface of the display apparatus may further be provided with operable components (not shown in the figure) for selecting distortion correction or distortion processing modes, which may adopt various forms such as radio buttons and dialog boxes. This allows operators to select performing distortion correction on endoscopic images, performing distortion processing on cut three-dimensional images according to pre-calibrated endoscopic camera parameters, or simultaneously performing endoscopic image distortion correction and three-dimensional image distortion processing to match distortion degrees therebetween. The display interface may also be provided with operable components (not shown in the figure) for selecting image fusion modes such as radio buttons, check boxes and dialog boxes, enabling operators to flexibly select the first image fusion mode, the second image fusion mode, or simultaneously display images obtained by both modes. Those skilled in the art shall appreciate that the steps of the method or algorithm described herein may be implemented directly by hardware, software modules executed by a processor, or a combination thereof. Software modules may be stored in random access memory (RAM), internal memory, read-only memory (ROM), electrically programmable ROM, electrically erasable programmable ROM, registers, hard disks, removable magnetic disks, CD-ROMs, or any other form of storage medium well known in the technical field.

The above embodiments may be implemented entirely or partially by software, hardware, firmware, or any combination thereof. When implemented by software, the embodiments may be implemented entirely or partially in the form of a computer program. The computer program includes one or more computer instructions. When the computer program instructions are loaded and executed on a computer, all or part of the processes or functions described in the present invention are realized. The computer may be a general-purpose computer, a dedicated computer, a computer network, or other programmable devices. The computer instructions may be stored in a computer-readable storage medium or transmitted from one computer-readable storage medium to another computer-readable storage medium via wired (e.g., coaxial cable, optical fiber, digital subscriber line) or wireless (e.g., infrared, wireless, microwave) means such as a website, server or data center. A computer-readable storage medium may be any available medium accessible by a computer, or an integrated server or data center including one or more available media. Available media include magnetic media (e.g., floppy disks, hard disks, magnetic tapes), optical media (e.g., DVD discs), semiconductor media (e.g., solid state disks, SSDs), and the like.

As described above, the navigation system in this embodiment includes a control device 2 and a display apparatus 3. Control device 2 includes a memory, a processor, and a computer program stored in the memory and executable on the processor; the processor executes the navigation method described above and implements the display step of the navigation method via display apparatus 3. Those skilled in the art shall appreciate that the memory of the control device may include random access memory (RAM) and non-volatile memory (NVM), such as at least one magnetic disk storage. Optionally, the memory may also include at least one storage device independent of the processor.

The processor of the control device may be a general-purpose processor, including a central processing unit (CPU), a network processor (NP), and the like; the processor may also be a digital signal processor (DSP), an application specific integrated circuit (ASIC), a field programmable gate array (FPGA), other programmable logic devices, discrete gate or transistor logic devices, or discrete hardware components.

Although certain embodiments of the present invention have been illustrated and described, it shall be apparent to those skilled in the art that various modifications and variations may be made without departing from the principles and spirit of the general inventive concept of the present invention, and the scope of the present invention shall be defined by the claims and equivalents thereof.

## Claims

1. A navigation method for spinal endoscopic surgery, wherein the method comprises the following steps:
a three-dimensional image acquisition step: acquiring a three-dimensional image of at least a part of a spine;
an image acquisition step: acquiring an endoscopic image in real time;
an orientation acquisition step: acquiring position and direction of a distal end of an endoscope in real time;
a three-dimensional image cutting step: cutting the three-dimensional image according to the position and direction of the endoscope distal end;
a fusion step: fusing the cut three-dimensional image with the endoscopic image; and
a display step: displaying a fused image.

2. The method according to Claim 1, wherein in the three-dimensional image cutting step, the three-dimensional image is cut into a cylindrical shape, wherein diameter of the cylinder is determined according to input from an operator, so as to define a cutting range of the three-dimensional image and obtain the cut three-dimensional image within a display range needed by the operator.

3. The method according to Claim 2, wherein the fusion step further comprises scaling a size of the endoscopic image correspondingly to three-dimensional images with different cutting ranges, and then performing the fusion.

4. The method according to Claim 1, wherein the direction of the endoscope distal end is an optical axis direction of the endoscope camera, wherein the cutting starts from the position of the focal plane of the endoscope camera, extends along the optical axis direction, and penetrates an entire depth of the three-dimensional image.

5. The method according to one of Claims 1 to 4, wherein in the three-dimensional image acquisition step, a preoperative three-dimensional image of at least a part of a spine is registered with a patient's pose under a navigation coordinate system.

6. The method according to one of Claims 1 to 5, further comprising a distortion processing step prior to the fusion step, wherein, in the distortion processing step, one of the following three modes is selectable according to input from an operator:
performing distortion correction on the acquired endoscopic image to at least partially eliminate a distortion effect of the endoscopic camera, such that the distortion-corrected endoscopic image matches the cut three-dimensional image; or
performing distortion processing on the cut three-dimensional image according to parameters of the endoscopic camera, such that the distortion-processed three-dimensional image matches the real-time acquired endoscopic image; or
performing distortion correction on the endoscopic image and distortion processing on the three-dimensional image simultaneously, such that distortion degrees of both are matched;
correspondingly, in the fusion step:
fusing the distortion-corrected endoscopic image with the cut three-dimensional image; or
fusing the real-time acquired endoscopic image with the distortion-processed three-dimensional image; or fusing the distortion-corrected endoscopic image with the distortion-processed three-dimensional image.

7. The method according to one of Claims 1 to 6, wherein, in the fusion step, one of the following two fusion modes is selectable or images obtained by the following two fusion modes are provided simultaneously according to input from an operator:
performing fusion in a mode that the cut three-dimensional image is overlaid underneath the real-time acquired endoscopic image; and
performing fusion by embedding the real-time acquired endoscopic image into the three-dimensional image acquired in the image acquisition step in a mode corresponding to the position and direction of the endoscope distal end.

8. The method according to one of Claims 1 to 7, wherein in the display step, a part of the three-dimensional image located behind the endoscope distal end is blurred and displayed.

9. The method according to one of Claims 1 to 8, further comprising an endoscope distal end calibration step of calibrating a positional relationship between the endoscope distal end and a first tracker disposed on the endoscope, wherein the endoscope distal end calibration step is implemented by means of a calibration tool without acquiring any image via the endoscope.

10. A computer-readable storage medium having a computer program stored thereon, wherein the computer program, when executed by a processor, implements the steps of the method according to one of Claims 1 to 9.

11. A control apparatus, comprising a memory, a processor, and a computer program stored in the memory and executable by the processor, wherein the processor executes the computer program to implement the steps of the method according to one of Claims 1 to 9.

12. An electronic device for navigation of spinal endoscopic surgery, wherein the electronic device comprises a display apparatus (3) and a processor, the processor being provided with a data interface;
wherein the data interface is connectable to an endoscope, such that the processor is capable of acquiring an endoscopic image in real time and acquiring a position and direction of an endoscope distal end in real time;
wherein the data interface further enables the processor to acquire a three-dimensional image of at least a part of a spine;
wherein when the processor is operating, a fused image is displayed on the display apparatus (3), and the fused image is obtained by fusing a three-dimensional image corresponding to the position and the direction of the endoscope distal end with the real-time acquired endoscopic image.

13. The electronic device according to Claim 12, wherein a display interface of the display apparatus (3) is provided with an input component; a display range of the three-dimensional image in the fused image is determined according to input from an operator via the input component.

14. The electronic device according to Claim 13, wherein the input component comprises a zoom icon for adjusting the range of the three-dimensional image, a component for an operator to select different display ranges of the three-dimensional image, or a component for an operator to input a numerical value relating to the display range of the three-dimensional image.

15. The electronic device according to Claim 13 or 14, wherein size of the endoscopic image in the fused image displayed on the display apparatus (3) is scaled correspondingly to the display range of the three-dimensional image.

16. The electronic device according to one of Claims 12 to 15, wherein the endoscopic image in the fused image displayed on the display apparatus (3) is a distortion-corrected image, the distortion correction at least partially eliminating a distortion effect of the endoscopic camera to make the distortion-corrected endoscopic image match the three-dimensional image; or the three-dimensional image in the fused image displayed on the display apparatus (3) is a distortion-processed three-dimensional image, the distortion processing being performed according to parameters of the endoscopic camera to make the distortion-processed three-dimensional image match the real-time acquired endoscopic image; or
the endoscopic image in the fused image displayed on the display apparatus (3) is a distortion-corrected image and the three-dimensional image is a distortion-processed three-dimensional image, with distortion degrees thereof being matched with each other.

17. The electronic device according to Claim 16, wherein the display apparatus (3) is further provided with an operation component for an operator to select to perform the distortion correction, the distortion processing, or both simultaneously.

18. The electronic device according to one of Claims 12 to 17, wherein in the fused image displayed on the display apparatus (3), a part of the three-dimensional image located behind the endoscope distal end is blurred.

19. The electronic device according to one of Claims 12 to 18, wherein a display interface of the display apparatus further displays one or more of a view on a fitted two-dimensional perspective section, a sagittal section view, a coronal section view, and an axial section view derived from the acquired three-dimensional image, each of the views presents a spatial positional relationship of the endoscope and a field angle thereof relative to anatomical structures of a patient.

20. A navigation system for spinal endoscopic surgery, wherein the navigation system comprises:
a tracking device (1), the tracking device (1) being configured to track a first tracker (4) disposed on an endoscope and a second tracker (5) disposed at a target site of a patient;
a display apparatus (3) and a processor, the processor being connected to the tracking device (1) and the endoscope; wherein the processor executes the method according to one of Claims 1 to 9 during operation, and implements display steps of the method via the display apparatus (3).

21. The navigation system according to Claim 20, further comprising one or more of the endoscopes, the first tracker and the second tracker.

22. The navigation system according to Claim 20 or 21, further comprising a calibration tool for calibrating position of the endoscope distal end relative to the first tracker, wherein a plurality of calibration holes are formed on the calibration tool, the plurality of calibration holes being provided with bottom surfaces having different inclination angles and/or different hole diameters, so as to respectively calibrate endoscopes having different inclination angles of distal sloped surface and/or different tube diameters.

23. A navigation system for spinal endoscopic surgery, wherein the navigation system comprises:
a tracking device (1), the tracking device (1) being configured to track a first tracker (4) disposed on an endoscope and a second tracker (5) disposed at a target site of a patient; and the electronic device according to one of Claims 12 to 19, wherein a processor of the electronic device is connected to the tracking device (1).

24. The navigation system according to Claim 23, further comprising one or more of the endoscopes, the first tracker and the second tracker.

25. The navigation system according to Claim 23 or 24, further comprising a calibration tool for calibrating position of the endoscope distal end relative to the first tracker, wherein a plurality of calibration holes are formed on the calibration tool, the plurality of calibration holes being provided with bottom surfaces having different inclination angles and/or different hole diameters, so as to respectively calibrate endoscopes having different inclination angles of distal sloped surface and/or different tube diameters.
